# EUROPÄISCHE PATENTSCHRIFT

(11) **EP 1 452 137 B1**
(45) Veröffentlichungstag und Bekanntmachung des Hinweises auf die Patenterteilung: **30.11.2005**
(21) Anmeldenummer: 04102217.9
(22) Anmeldetag: 09.02.1998
(51) Int. Cl.: A61B 6/14

(54) **Verfahren und Einrichtung zur Erstellung von Röntgenaufnahmen von Körperteilen eines Menschen**
Method and apparatus for producing x-ray exposures of human body parts
Procedé et appareil pour produire des radiographies des parties du corps humain

(30) Priorität: 17.02.1997 DE 19706102
(43) Veröffentlichungstag der Anmeldung: 01.09.2004
(62) Teilanmeldung aus: 02006254.3
(73) Patentinhaber: Sirona Dental Systems GmbH, 64625 Bensheim (DE)
(72) Erfinder: Zeller, Uwe, 88400, Biberach (DE); Günther, Werner, 64625, Bensheim (DE); Schulze-Ganzlin, Ulrich, 64653, Lorsch (DE); Döbert, Michael, 64653, Lorsch (DE)
(74) Vertreter: Sommer, Peter

(56) Entgegenhaltungen:
- EP-A- 0 229 971
- EP-A- 0 632 994
- US-A- 4 188 537
- US-A- 5 600 699

## Beschreibung

### Technisches Gebiet

Die Erfindung bezieht sich auf ein Verfahren und eine Vorrichtung zur Erstellung von Röntgenaufnahmen von Körperteilen eines Menschen, insbesondere von Röntgen-Schichtaufnahmen vom Kiefer oder Schädel eines Patienten, bei dem ein von einer Strahlenquelle erzeugtes und durch eine Blendenöffnung einer Primärblende begrenztes Strahlenbündel nach Durchdringung des Aufnahmeobjektes auf eine Detektoranordnung trifft, die mindestens einen röntgenbilderfassenden Detektor aufweist.

### Stand der Technik

In der zahnärztlichen Röntgentechnik werden Verfahren und Vorrichtungen angewendet, mit denen es möglich ist, Schichtaufnahmen von einem Menschen, insbesondere vom Bereich des Kiefers, zu erstellen. Eine besondere Anwendung liegt in der Erstellung von Schichtaufnahmen, deren Schichtverlauf senkrecht zum Kieferbogen verläuft. Solche Schichtaufnahmen werden auch Transversalschnitte genannt. Im Vergleich zu den sonst üblichen Panorama-Schichtaufnahmen weisen solche Transversalschnitte einen besonders kleinen Tiefenschärfenbereich auf. In der EP-0 229 971 wird eine Vorrichtung, mit der solche Schichtaufnahmen auf einem Röntgenfilm möglich sind, aufgezeigt.

In der EP-0 632 994 wird eine Vorrichtung zur Erstellung von Röngenaufnahmen auf digitalem Weg beschrieben. Hierzu ist eine Zeilendetektorkamera mit einem Detektor vorgesehen, der als ein- oder mehrteiliger CCD-Sensor ausgeführt ist. Die Abmessungen der Detektoranordnung betragen, unabhängig davon, ob ein ein- oder mehrteiliger Sensor verwendet wird, typischerweise 135 bis 180 mm in der Bildhöhe und ca. 6 mm in der Bildbreite. Diese Maße berücksichtigen einerseits die für eine gute Diagnose notwendige Bilderfassungsgröße, andererseits eine ausreichende Tiefenschärfe bei Betrachtung der einzelnen Schichten. In der Praxis hat sich gezeigt, daß das Blenden- und Detektorsystem so abgestimmt sein muß, daß der nutzbare Strahlenfächer für transversale Schichten, also für die obengenannten Transversalschnitte, eine Breite von mindestens 20 mm in Detektorebene aufweisen muß, um einen Tiefenschärfenbereich von ca. 1 bis 3 mm erzielen zu können. Eine Detektoranordnung in der vorgenannten Größenordnung ist mit der heutigen Technologie vergleichsweise teuer.

Aus der US 4,188,537 ist eine dentale Röntgeneinrichtung bekannt, bei der ein sich in vertikaler Richtung erstreckender Zeilensensor oder ein einzelner Sensor zur Erstellung einer Röntgenaufnahme in vertikaler Richtung entlang einer Linie bewegt wird. Der Sensor kann dabei auch einer Drehbewegung folgen, so dass zusammen mit der vertikalen Bewegung der gesamte Bereich des Patienten durch Röntgenstrahlen erfaßt wird.

### Offenbarung der Erfindung

Ziel der vorliegenden Erfindung ist es, ein Verfahren und eine Vorrichtung zu schaffen, mit der es möglich ist, die eingangs erwähnten Schichtaufnahmen schneller durchführen zu können.

Die Aufgabe wird mit den Mitteln des Anspruchs 1 gelöst. Bei der erfindungsgemäßen Einrichtung wird in der Detektoranordnung ein CCD-Detektor verwendet, der nach dem Prinzip der Time Delay and Integration (TDI) arbeitet. Das Austaktregister ist dabei als Horizontalregister ausgebildet, wobei die TDI-Richtung quer zur Verschieberichtung des Detektors verläuft. Darüber hinaus ist im Detektor ein zweites Ausleseregister vorgesehen, wobei der Detektor in seiner Integrationsrichtung umschaltbar ausgebildet ist. Dies ermöglicht die Auslesung des Detektors in zwei Richtungen.

Das erfindungsgemäße Verfahren zur Erstellung von Röntgenaufnahmen unter Verwendung einer erfindungsgemäßen Einrichtung sieht vor, die Teilaufnahmen nicht nur in der Vorlaufphase, sondern auch in der Rücklaufphase der die Röntgenstrahlenquelle und die Detektoranordnung tragenden Dreheinheit durchzuführen. Der gesamte Aufnahmeablauf läßt sich so wesentlich beschleunigen.

### Kurze Beschreibung der Zeichnungen

Anhand der Zeichnung werden mehrere Ausführungsbeispiele der Erfindung näher beschrieben. Es zeigen:
- Fig. 1: ein zahnärztliches Röntgendiagnostikgerät mit der erfindungsgemäßen Vorrichtung in einer Seitenansicht,
- Fig. 2: eine Prinzipskizze zur Erläuterung der mechanischen Zusammenhänge der Vorrichtung,
- Fig. 3: eine Ausführungsform einer Detektoranordnung,
- Fig. 4: eine Version einer Detektoranordnung,
- Fig. 5: eine Prinzipskizze zur Erläuterung von Transversalschnitten und
- Fig. 6: ein Blockschaltbild.

### Ausführungsform(en) der Erfindung

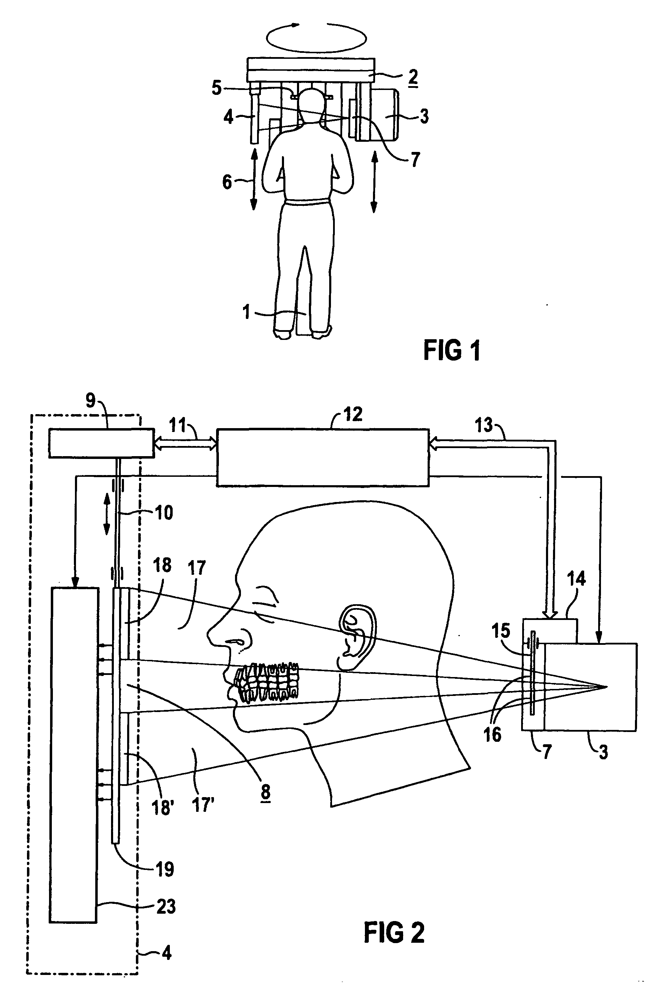

Die Fig. 1 zeigt in einer Prinzipdarstellung ein zahnärztliches Röntgendiagnostikgerät zur Erstellung von Panorama-Schichtaufnahmen, welches gemäß der Erfindung auch zur Darstellung von Transversalschnitten eingesetzt werden kann. Das Gerät enthält eine in der Höhe verstellbare Tragsäule 1, an der eine Dreheinheit 2 gehaltert ist, die Träger einerseits einer Röntgenstrahlenquelle 3 und andererseits diametral dazu einer Röntgen-Detektorkamera 4 ist. Mit 5 ist eine Kopfhalte- und Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte- und Positioniereinrichtung sind bekannt und beispielsweise in der eingangs genannten EP-0 632 994 beschrieben. Die Zeilenkamera 4 besteht aus einem länglichen Gehäuse mit einem nicht näher bezeichneten Schlitz an der der Strahlenquelle zugewandten Seite. Hinter dem Schlitz befindet sich im Innern der Kamera eine Detektoranordnung 8 mit einem oder mehreren strahlenempfindlichen Detektoren, z.B. in Form von CCD-Sensoren. Aufbau und Anordnung werden später noch näher erläutert. Die Detektoranordnung 8 ist innerhalb der Zeilendetektorkamera in Richtung ihrer Längsachse in Richtung des Pfeiles 6 verstellbar gehaltert. Im folgenden wird von einer Detektoranordnung mit zwei aktiven Detektorelementen ausgegangen. Diese Anordnung stellt nur eine von mehreren denkbaren, im Rahmen der Erfindung liegenden Ausführungsformen dar. Synchron dazu ist auch ein mit 7 bezeichnetes Blendensystem, welches die Primärblende beinhaltet, verstellbar gehaltert. Die elektromechanische Verbindung der Zeilendetektorkamera 4 mit dem Blendensystem 7 wird anhand der Fig. 2 näher erläutert.

Die im Innern angeordneten, später noch näher erläuterten Detektorelemente können mittels einer geeigneten Verstelleinrichtung, hier mittels eines Schrittmotors 9 und einer Spindel 10, entlang der Detektorhauptachse verstellt werden. Der Schrittmotor 9 kommuniziert über eine (serielle) Schnittstelle 11 mit einer Steuerelektronik 12 der Gerätesteuerung des Röntgengerätes. Die Steuerelektronik 12 gibt über eine weitere Schnittstelle 13 Steuerbefehle an einen an der Röntgenstrahlenquelle 3 angeordneten Stellantrieb 14. Mit diesem Stellantrieb erfolgt die synchrone Verstellung einer Primärblende 15 des Blendensystems 7. Die Primärblende 15 enthält zwei beabstandet angeordnete Blendenöffnungen 16. Die von der Strahlenquelle 3 erzeugten Röntgenstrahlen werden so in zwei Strahlenbündel 17, 17' unterteilt, die so fokussiert sind, daß sie exakt auf zwei im Innern der Zeilendetektorkamera 4 angeordnete Detektoren 18, 18' treffen. Die beiden Detektoren 18, 18' sind auf einem Träger 19 angeordnet, der, wie beschrieben, mittels der Verstelleinrichtung 9, 10 in der angegebenen Pfeilrichtung verstellbar ist.

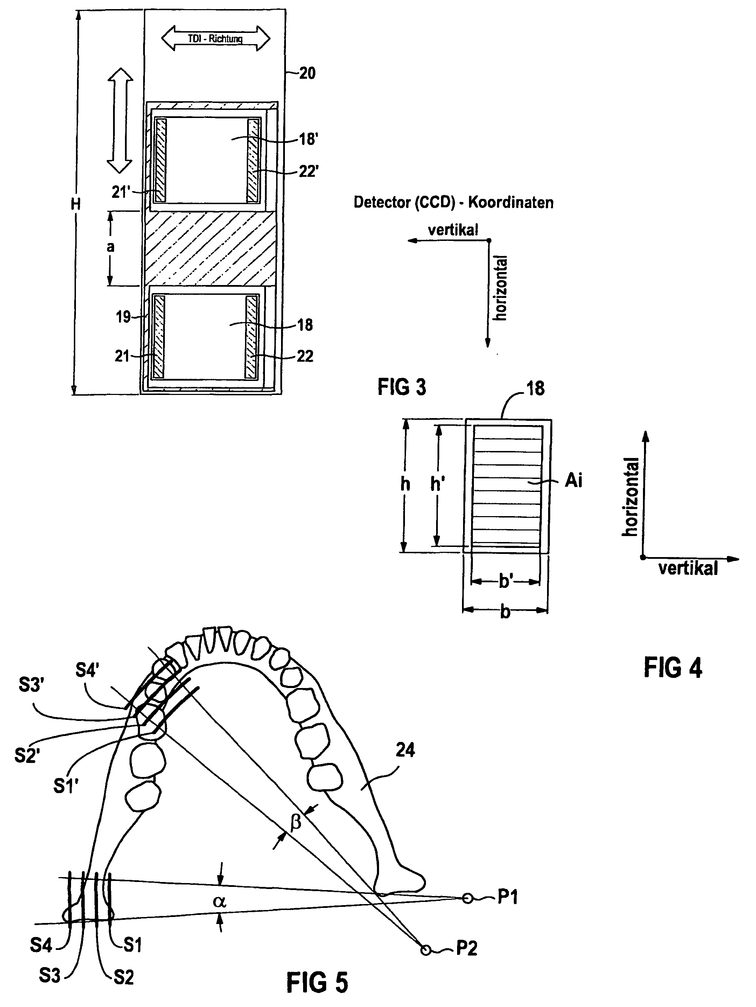

Die Fig. 3 zeigt die Detektoranordnung in einer Frontansicht. Der Träger 19, auf dem die beiden Detektoren 18, 18' 9 befestigt sind, ist in einem Rahmen 20 verschiebbar gehaltert. Mit 21 bzw. 21' sind die Austaktregister der Detektoren bezeichnet, die, wie aus der Koordinatenbezeichnung rechts im Bild ersichtlich, als Horizontalregister ausgebildet sind, d.h. die TDI-Richtung (TDI steht für Time Delay and Integration und ist eine CCD-spezifische Technologie) verläuft quer zur Verschieberichtung. Mit 22, 22' sind zweite Ausleseregister bezeichnet, die gemäß einer vorteilhaften Variante dann vorgesehen werden müssen, wenn man das Austakten in der entgegengesetzten Richtung ausführen will, wenn man also Teilaufnahmen während der Rücklaufphase der Dreheinheit machen will.

Mit 23 in Figur 2 ist die Ansteuerelektronik für die beiden Detektoren 18, 18' bezeichnet.

Fig. 4 zeigt einen einzelnen Detektor 18 mit den bei Intraoral-Sensoren üblichen Abmessungen. Solche Sensoren haben typischerweise eine Höhe (h) von 30 mm und eine Breite (b) von 20 mm. Die von Strahlung ausgeleuchtete Sensorfläche ist mit Ai bezeichnet und beträgt in der Höhe (h') 26 mm und in der Breite (b') 18 mm.

Anhand der Fig. 5 wird der Ablauf zur Erzielung von Transversalschichtaufnahmen näher erläutert. Es wird dabei davon ausgegangen, daß vier Transversalschnitte in den vier angegebenen Schichtebenen 1, 2, 3 und 4 aufgenommen werden sollen.

Die Bedienperson wählt zunächst die Aufnahmeparameter (untersuchter Bereich, Anzahl, Qualität und Größe der Transversalschnitte sowie Dosis). Diese Parameter können am Röntgengerät oder an einem angeschlossenen PC durch Auswahl vordefinierter Programme oder auch durch manuelles Zusammenstellen der Parameter erfolgen. Nach Positionierung des Patienten in der Kopfhalte- und Positoniereinrichtung 5 (Fig. 1) wird der Aufnahmeablauf gestartet. Das Gerät justiert sich zunächst durch Anfahren von Referenzpunkten und positioniert sich anschließend in einer Startposition für die nachfolgende Aufnahmeserie. Die Anzahl der Einzelaufnahmen ergibt sich durch die Anzahl der Transversal-Schichtaufnahmen und die Anzahl der Aufnahmephasen. Im Ausführungsbeispiel nach Fig. 5 sei angenommen, daß von dem aufgezeichneten Kieferbogen 23 zunächst vier verschiedene Schichtaufnahmen S1 bis S4 vom linken Kiefergelenk und danach vier weitere Schichtaufnahmen S1' bis S4' von einem weiteren Kieferabschnitt erstellt werden sollen. Die Dreheinheit 2 wird zunächst in die Position P1 gefahren, von der aus von der Strahlenquelle Strahlen auf den gewünschten Abbildungsbereich abgegeben werden können, der beispielsweise den eingezeichneten Winkel α umfassen soll. Die Schichtaufnahme S1 wird erstellt, indem beginnend von einer Ausgangsposition über den Winkel α gestrahlt wird. Nachdem die Aufnahme für die Schichtlage S1 erstellt ist, wird die Strahlenquelle abgeschaltet und die Dreheinheit um den Schwenkwinkel α wieder zurück in die Ausgangsposition gefahren. Danach werden in der Folge die Schichtlagen S2, S3 und S4 erstellt. Andere Schichtlagen für weitere Objektausschnitte können analog erstellt werden, beispielsweise wie dargestellt, von der Position P2 aus. Der kinematische Bewegungsablauf der die Strahlenquelle und Zeilendetektorkamera aufnehmenden Dreheinheit ist an sich bekannt, ebenso die dazu erforderliche Ansteuerung der Detektoren nach dem TDI-Verfahren, um die gewünschte Schichtung zu erhalten.

Bei der zuletzt beschriebenen Aufnahmeprozedur wird die eigentliche Aufnahme mit Strahlung während des Vorlaufs der Dreheinheit durchgeführt. In der Rücklaufphase ist die Strahlung abgeschaltet oder sie wird durch eine Blendenverstellung unwirksam gemacht.

Um die vier Schichten im Ausführungsbeispiel erfassen zu können, gibt es mehrere Möglichkeiten. Die eine besteht darin, zunächst eine Schicht komplett zu erstellen, d.h. bei einer Detektoranordnung mit zwei Detektoren zunächst zwei Aufnahmesequenzen mit Detektorverschiebung durchzuführen und entsprechend danach die weiteren Schichten S2, S3 und S4 aufzunehmen.

Gemäß der Erfindung werden in einer Detektorposition alle vier Schichten aufgenommen, danach wird die Detektoranordnung verschoben und anschließend werden wiederum alle vier Schichten in der zweiten Detektoranordnung aufgenommen. Diese Version hat den Vorteil, daß weniger Bewegungsvorgänge notwendig sind, die unter Umständen den gesamten Ablauf beschleunigen.

Wie eingangs bereits angesprochen, kann es vorteilhaft sein, während des Rücklaufes der Dreheinheit Schichtaufnahmen durchzuführen. Dazu ist es notwendig, die Detektoren mit einem zweiten Ausleseregister (Pos. 22, 22' in Fig. 3) auszustatten, um den TDI-Prozeß auch in der entgegengesetzten Richtung ausführen zu können.

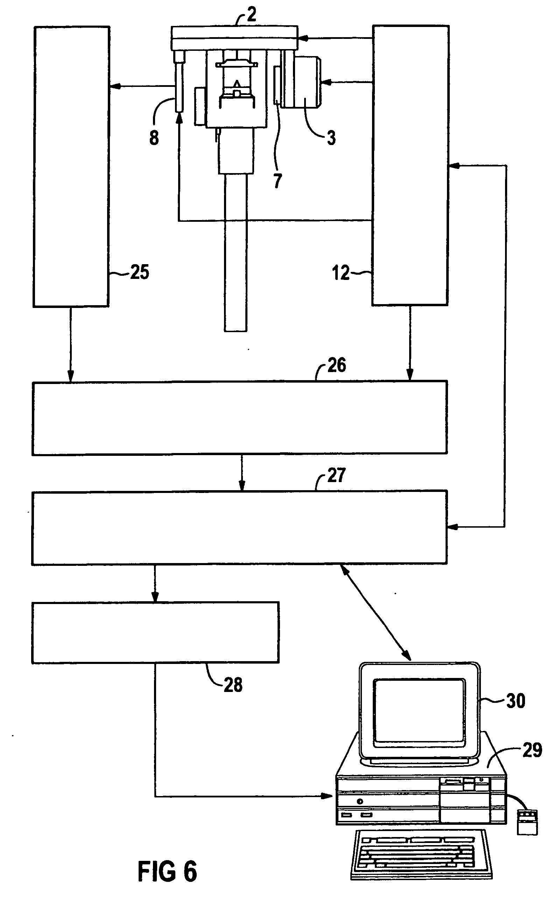

Die Fig. 6 zeigt ein Blockschaltbild der erfindungsgemäßen Vorrichtung und verdeutlicht die Beziehung zwischen den einzelnen Komponenten. Die Gerätesteuerung 12 erzeugt Steuersignale für die Detektoranordnung 8, das Blendensystem 7 und Röntgenquelle 3 und koordiniert für jede Teilbildsequenz den Ablauf bzw. die Bewegung der Detektoranordnung 8 und des Strahlers 3 entsprechend der vorbestimmten Bahn bei einer Aufnahme. Die während der Sequenz erfaßten Bilddaten werden in einen Zwischenspeicher 25 abgelegt. Wie durch die Pfeilangaben erkennbar, steuert die Gerätesteuerungselektronik 12 nicht nur den Strahler 3, das Blendensystem 7 und die Detektoranordnung 8, sondern liefert gleichsam aufnahmebezogene Strahlungs-, Positions- und Bahndaten an eine Software 26. Diese verarbeitet auch die in dem Zwischenspeicher 25 abgelegten Bilddaten der Teilbilder, die mit den Detektoren gewonnen worden sind, zu einem 'Rohbild'. Dieses 'Rohbild' wird anschließend in einer weiteren Software 27 zu einem Röntgenbild verarbeitet, welches danach in einer Datenbank 28 abgelegt wird. Von dort aus kann es beispielsweise über einen PC 29 abgerufen und auf einem Monitor 30 dargestellt werden. Vom PC 29 können vorteilhafterweise durch die für eine Aufnahme notwendigen Parameter aus der Software 27 generiert werden, die von dort aus an die Gerätesteuerung weitergeleitet werden.

Die zur Erzeugung der Schichtbilder notwendige Summation bzw. Integration kann - wie aufgezeigt - mit Hilfe der CCD, die im sog. TDI-Modus beschrieben werden, erfolgen. Hierbei entsteht das Bild während der Datenerfassung durch eine 'analoge' Summation innerhalb der aktiven CCD-Fläche.

Wie eingangs erwähnt, sollte die Detektorbreite mindestens 20 mm aufweisen, um Mindest-Anforderungen an die Tiefenschärfe der aufzulösenden Transversalschicht zu erfüllen. Durch Querverschieben der vorgenannten Detektorstrukturen und durch Wiederholung der beschriebenen Aufnahmeprozeduren kann die wirksame Detektorbreite vergrößert und somit die Tiefenschärfe der Transversalschicht verbessert werden.

Mit jeder Querverschiebung entsteht ein weiterer Aufnahmesatz. Diese Aufnahmesätze können, wie beschrieben, mit dem "analogen" TDI-Verfahren oder "digitale" Summation erstellt werden. Um die Querverschiebung mit zu berücksichtigen, müssen diese Aufnahmesätze ebenfalls aufsummiert werden. Zur Summation der digitalisierten Aufnahmesätze ist verständlicherweise nur das "digitale" Verfahren sinnvoll anwendbar.

Wie bereits erwähnt, erfolgt die Primärblendeneinstellung synchron zu der Verstellung der Detektoren. Dies kann durch eine motorische verschiebbare Schablone in der in Fig. 2 dargestellten Weise erfolgen. Alternativ kann auch die Blende durch eine entsprechende Verstellung einer unteren und oberen Abgrenzung erfolgen; ebenso ist es denkbar, eine drehbare Blende mit an unterschiedlichen Stellen befindlichen Blendenöffnungen vorzusehen.

## Patentansprüche

1. Einrichtung zur Erstellung von Röntgenaufnahmen in Form mehrerer Transversalschichtaufnahmen von Körperteilen eines Menschen, insbesondere von Röntgen-Schichtaufnahmen vom Kiefer oder Schädel eines Patienten, aufweisend eine Strahlenquelle (3), eine Blendenöffnung (16) einer Primärblende (15) und eine Detektoranordnung (8) mit mindestens einem Detektor (18, 18'), wobei die Detektoranordnung (8) in der Folge entlang der Längsachse und/oder Querachse der Detektorfläche verschoben werden kann und dazu die Blendenöffnung (16,16') der Primärblende (15) entsprechend angepaßt wird und wobei der Detektor (18,18') nach dem Prinzip der Time Delay and Integration (TDI) arbeitet und ein als Horizontalregister ausgebildetes Austaktregister (21) umfasst, wobei die TDI-Richtung quer zur Verschieberichtung des Detektors verläuft, **dadurch gekennzeichnet, dass** ein im Detektor (18,18') zweites Ausleseregister (22) vorgesehen ist und der verwendete Detektor (18; 18') in seiner Integrationsrichtung umschaltbar ausgebildet ist.

2. Verfahren zur Erstellung von Röntgenaufnahmen zur Erzielung mehrerer Transversalschichtaufnahmen, wobei die strahlungsempfindliche Fläche (Ai) des Detektors (18, 18') eine Teilfläche der zur Objektaufnahme erforderlichen Detektorfläche (Ages.) beträgt und wobei die Bildaufnahme in mehreren, zeitlich getrennten Abschnitten erfolgt, indem nach einer ersten Teilaufnahme die Detektoranordnung (8) in der Folge entlang der Längsachse und/oder Querachse der Detektorfläche verschoben wird und dazu die Blendenöffnung (16, 16') der Primärblende (15) entsprechend angepaßt wird, **dadurch gekennzeichnet, daß** eine Einrichtung nach Anspruch 1 verwendet wird, wobei die Teilaufnahmen in einer Vorlaufphase und/oder in einer Rücklaufphase einer die Röntgenstrahlenquelle (3) und die Detektoranordnung (8) tragenden Dreheinheit (2) durchgeführt werden.

## Claims

1. A system for the acquisition of X-ray images in the form of a plurality of transversal tomograms of parts of the body of a human being, particularly tomograms of the jaw or skull of a patient, comprising a source of radiation (3), an aperture (16) of a primary diaphragm (15), and a detector system (8) having at least one detector (18, 18'), in which said detector system (8) can be subsequently shifted along the longitudinal axis and/or transverse axis of the detector surface, for which purpose said aperture (16, 16') of said primary diaphragm (15) is appropriately adjusted, and in which said detector (18, 18') operates on the principle of Time Delay and Integration (TDI) and comprises a clock out register (21) in the form of a horizontal register, the TDI direction running at right angles to the direction of shift of said detector, wherein a second read out register (22) is provided in said detector (18, 18') and the detector (18, 18') used is adapted for change-over switching of its integration direction.

2. A method for the acquisition of X-ray images to achieve a plurality of transversal tomograms, in which the radiosensitive surface (Ai) of said detector (18, 18') is a subarea of the detector surface necessary for registering the object (Ages.) and in which imaging is effected in a number of temporally separate phases in that, following a first partial image, the detector system (8) is subsequently displaced along the longitudinal axis and/or transverse axis of the detector surface, for which purpose said aperture (16, 16') of said primary diaphragm (15) is appropriately adjusted, wherein a system as defined in claim 1 is used, in which the partial images are acquired in a forward phase and/or in a reverse phase of a rotatable unit (2) supporting said X-ray source (3) and said detector system (8).

## Revendications

1. Dispositif pour produire des radiographies sous la forme de plusieurs radiographies de couches transversales de parties du corps d'un être humain, en particulier des tomographies de la mâchoire ou du crâne d'un patient, présentant une source de rayons (3), une ouverture de diaphragme (16) d'un diaphragme primaire (15) et un dispositif de détection (8) ayant au moins un détecteur (18, 18'), ledit dispositif de détection (8) pouvant être déplacé dans la suite le long de l'axe longitudinal et/ou de l'axe transversal de la surface de détecteur et l'ouverture de diaphragme (16, 16') du diaphragme primaire (15) étant à cet effet adaptée de manière correspondante, et ledit détecteur (18, 18') travaillant selon le principe Time Delay and Integration (TDI) et comprenant un registre de synchronisation (21) réalisé en tant que registre horizontal, la direction TDI s'étendant transversalement à la direction de déplacement du détecteur, **caractérisé par le fait qu'**un deuxième registre de lecture (22) est prévu dans le détecteur (18, 18') et que le détecteur (18, 18') utilisé est réalisé de manière à ce que sa direction d'intégration puisse être commutée.

2. Procédé pour produire des radiographies afin d'obtenir plusieurs radiographies de couches transversales, dans lequel la surface sensible au rayonnement (Ai) du détecteur (18, 18') représente une surface partielle de la surface du détecteur (Ages) nécessaire à la radiographie de l'objet, et dans lequel la radiographie s'effectue en plusieurs étapes séparées dans le temps en déplaçant, après une première radiographie partielle, le dispositif de détection (8) dans la suite le long de l'axe longitudinal et/ou de l'axe transversal de la surface de détecteur et en adaptant, à cet effet, l'ouverture de diaphragme (16, 16') du diaphragme primaire (15) de manière correspondante, **caractérisé par le fait que** l'on utilise un dispositif selon la revendication 1, les radiographies partielles étant réalisées dans une phase d'avance et/ou dans une phase de retour d'une unité de rotation (2) qui supporte la source de rayons X (3) et le dispositif de détection (8).
